# EUROPEAN PATENT APPLICATION

(11) **EP 4 796 019 A1**
(43) Date of publication of application: **26.08.2026**
(21) Application number: 24890634.9
(22) Date of filing: 11.11.2024
(51) Int. Cl.: A61B 1/00, A61B 1/04

(54) **METHOD AND DEVICE FOR DETECTING EXCRETION OF CAPSULE ENDOSCOPE, AND CAPSULE ENDOSCOPE**

(30) Priority: 13.11.2023 CN 202311506599
(71) Applicant: Guangzhou Side Medical Technology Co., Ltd., Guangzhou, Guangdong 510700 (CN)
(72) Inventor: CHEN, Huanfeng, Guangzhou, Guangdong 510700 (CN); LIU, Side, Guangzhou, Guangdong 510700 (CN); WANG, Yunzhong, Guangzhou, Guangdong 510700 (CN)
(74) Representative: Vogelbruch, Keang
(86) International application number: PCT/CN2024/131375
(87) International publication number: WO 2025/103279

(57) **Abstract**

Provided are a method and device for detecting the excretion of a capsule endoscope, and a capsule endoscope. The method comprises: in a retention detection mode, turning off power supplies for other modules except for a counter, a clock signal generator, and a register in the capsule endoscope, and intermittently awakening a radio frequency transmitting circuit to transmit an instruction, wherein the instruction is pre-stored in the register, in each awakening process, triggering the counter to perform counting according to a clock signal generated by the clock signal generator, and when the counter overflows, awakening the radio frequency transmitting circuit to transmit the instruction; and determining whether the capsule endoscope is retained in the body of an object of endoscope examination or not according to whether a data recorder receives the instruction or not.

## Description

### CROSS-REFERENCE TO RELATED APPLICATIONS

The present invention claims the priority to the Chinese patent application with the filing No. 202311506599.1 filed with the Chinese Patent Office on November 13, 2023, and entitled "METHOD AND DEVICE FOR DETECTING EXCRETION OF CAPSULE ENDOSCOPE, AND CAPSULE ENDOSCOPE", which is incorporated herein by reference in entirety.

### TECHNICAL FIELD

The present invention relates to the technical field of capsule endoscope detection, and in particular, to a method for detecting excretion of a capsule endoscope, an apparatus, a capsule endoscope, a storage medium, and a computer program product.

### BACKGROUND ART

After completing a digestive tract examination, a capsule endoscope may be retained in the examinee's body and cannot be excreted. Thus, it is necessary to detect whether the capsule endoscope is retained in the examinee's body.

In existing methods for detecting excretion of a capsule endoscope, after the capsule endoscope completes a digestive tract examination, it enters a sleep mode, an external data recorder sends a wake-up signal at regular intervals, and upon receiving the wake-up signal, the capsule endoscope sends a real-time image, and the external data recorder determines whether the capsule endoscope is retained in the examinee's body. In this method, a radio-frequency receiving circuit needs to operate continuously, and sending an image requires the image sensor chip, the main control chip, and other components to work together, resulting in high power consumption.

Existing methods for detecting excretion of a capsule endoscope have the drawback of high power consumption, making it difficult to support long operating duration of the capsule endoscope, which leads to a low detection rate of capsule endoscope retention.

### SUMMARY

A method for detecting excretion of a capsule endoscope is provided, wherein the capsule endoscope includes a counter, a radio-frequency transmission circuit, a clock signal generator, and a register; and the method includes: in a retention detection mode, powering off other modules in the capsule endoscope except the counter, the clock signal generator, and the register, and intermittently waking up the radio-frequency transmission circuit to issue a command, wherein the command is pre-stored in the register; triggering the counter to count according to a clock signal generated by the clock signal generator during each wake-up process, and waking up the radio-frequency transmission circuit to issue the command when the counter overflows; and determining that the capsule endoscope is retained in an examinee's body in response to a data recorder receiving the command.

An apparatus of detecting excretion of a capsule endoscope is provided, wherein the capsule endoscope includes a counter, a radio-frequency transmission circuit, a clock signal generator, and a register; and the apparatus includes: a command issuing module, configured to in a retention detection mode, power off other modules in the capsule endoscope except the counter, the clock signal generator, and the register, and intermittently wake up the radio-frequency transmission circuit to issue a command, wherein the command is pre-stored in the register; a wake-up module, configured to trigger the counter to count according to a clock signal generated by the clock signal generator during each wake-up process, and wake up the radio-frequency transmission circuit to issue the command when the counter overflows; and a retention determination module, configured to determine that the capsule endoscope is retained in an examinee's body in response to a data recorder receiving the command.

A capsule endoscope includes a counter, a radio-frequency transmission circuit, a clock signal generator, and a register, wherein the register is configured to store a command; the clock signal generator is configured to generate a clock signal; the counter is configured to count, wherein in a retention detection mode, other modules in the capsule endoscope except the counter, the clock signal generator, and the register is powered off, and the radio-frequency transmission circuit is intermittently woken up to issue a command, wherein the radio-frequency transmission circuit is woken up when the counter overflows.

A computer-readable storage medium stores a computer program which, when executed by a processor, causes the processor to perform the steps of the method described above.

A computer program product comprises a computer program which, when executed by a processor, causes the processor to perform the steps of the method described above.

### BRIEF DESCRIPTION OF DRAWINGS

To more clearly illustrate the technical solutions in the embodiments of the present invention or in the related art, the following briefly introduces the drawings required for describing the embodiments or the related art. Apparently, the drawings described below are only some embodiments of the present invention, and those of ordinary skill in the art can obtain other drawings based on these drawings without inventive efforts.
FIG. 1 is a diagram of an application environment of a method for detecting excretion of a capsule endoscope according to an embodiment;
FIG. 2 is a schematic flowchart of a method for detecting excretion of a capsule endoscope according to an embodiment;
FIG. 3 is an internal structural diagram of a capsule endoscope according to an embodiment;
FIG. 4 is a schematic flowchart of a method for detecting excretion of a capsule endoscope according to another embodiment; and
FIG. 5 is a structural block diagram of an apparatus of detecting excretion of a capsule endoscope according to an embodiment.

### DETAILED DESCRIPTION OF EMBODIMENTS

To make the objectives, technical solutions, and advantages of the present invention more clearly understood, the present invention is further described in detail below with reference to the drawings and embodiments. It should be understood that the specific embodiments described herein are only used to explain the present invention and are not intended to limit the present invention.

Reference to "embodiment" in the present invention means that a particular feature, structure, or characteristic described in connection with the embodiment can be included in at least one embodiment of the present invention. The appearances of this phrase in various places in the specification do not necessarily all refer to the same embodiment, nor do they refer to independent or alternative embodiments that are mutually exclusive with other embodiments. It is explicitly and implicitly understood by those skilled in the art that the embodiments described in the present invention can be combined with other embodiments.

The present invention provides a method for detecting excretion of a capsule endoscope, an apparatus, a capsule endoscope, a computer-readable storage medium, and a computer program product.

In a first aspect, the present invention provides a method for detecting excretion of a capsule endoscope, the capsule endoscope including a counter, a radio-frequency transmission circuit, a clock signal generator, and a register; and the method includes:
in a retention detection mode, powering off other modules in the capsule endoscope except the counter, the clock signal generator, and the register, and intermittently waking up the radio-frequency transmission circuit to issue a command, wherein the command is pre-stored in the register;
triggering the counter to count according to a clock signal generated by the clock signal generator during each wake-up process, and waking up the radio-frequency transmission circuit to issue the command when the counter overflows; and
determining that the capsule endoscope is retained in an examinee's body in response to a data recorder receiving the command.

In one or more embodiments, before a step of in a retention detection mode, powering off other modules in the capsule endoscope except the counter, the clock signal generator, and the register, and intermittently waking up the radio-frequency transmission circuit to issue a command, the method further includes:
determining whether a user issued endoscopic examination completion command is received; and
entering the retention detection mode in response to receiving a user issued endoscopic examination completion command.

In one or more embodiments, before a step of in a retention detection mode, powering off other modules in the capsule endoscope except the counter, the clock signal generator, and the register, and intermittently waking up the radio-frequency transmission circuit to issue a command, the method further includes:
acquiring a current image captured by a camera;
inputting the current image into a pre-trained model to obtain a detection result; and
entering the retention detection mode when the detection result indicates whether the endoscopic examination is completed.

In one or more embodiments, before a step of in the retention detection mode, powering off other modules in the capsule endoscope except the counter, the clock signal generator, and the register, and intermittently waking up the radio-frequency transmission circuit to issue a command, the method further includes:
determining whether a wake-up duration of the capsule endoscope has reached a preset duration; and
entering the retention detection mode in response to a wake-up duration of the capsule endoscope reaching a preset duration.

In one or more embodiments, the step of triggering the counter to count according to a clock signal generated by the clock signal generator includes:
triggering the counter to count cyclically according to the clock signal generated by the clock signal generator, wherein the number of counting cycles is configured to determine whether the counter overflows; and
triggering the counter to count according to the clock signal generated by the clock signal generator during each counting operation in a cyclic process, and resetting the count value for a next counting cycle when the count of the counter reaches a preset value.

In one or more embodiments, after waking up the radio-frequency transmission circuit, the method further includes:
turning off the counter after the radio-frequency transmission circuit is woken up; and
triggering the counter to resume counting after the command is issued.

In one or more embodiments, the step of determining that the capsule endoscope is retained in an examinee's body in response to a data recorder receiving the command includes:
determining that the capsule endoscope is retained in the examinee's body when the data recorder receives the command; or
determining that the capsule endoscope has been excreted from the examinee's body when the data recorder does not receive the command within a preset time duration.

In one or more embodiments, after determining that the capsule endoscope is retained in an examinee's body in response to a data recorder receiving the command, the method further includes:
waking up an image sensor and a main control chip of the capsule endoscope to capture an image when determining that the capsule endoscope is retained in the examinee's body; and
sending the image to the data recorder, wherein the image is configured to determine a cause of retention of the capsule endoscope.

In a second aspect, the present invention further provides an apparatus of detecting excretion of a capsule endoscope, wherein the capsule endoscope includes a counter, a radio-frequency transmission circuit, a clock signal generator, and a register; and the apparatus includes:
a command issuing module, configured to in a retention detection mode, power off other modules in the capsule endoscope except the counter, the clock signal generator, and the register, and intermittently wake up the radio-frequency transmission circuit to issue a command, wherein the command is pre-stored in the register;
a wake-up module, configured to trigger the counter to count according to a clock signal generated by the clock signal generator during each wake-up process, and wake up the radio-frequency transmission circuit to issue the command when the counter overflows; and
a retention determination module, configured to determine that the capsule endoscope is retained in an examinee's body in response to a data recorder receiving the command.

In one or more embodiments, the apparatus further includes a mode determination module, configured to determine whether a user issued endoscopic examination completion command is received and enter the retention detection mode in response to receiving a user issued endoscopic examination completion command.

In one or more embodiments, the apparatus further includes a mode determination module, configured to acquire a current image captured by a camera; input the current image into a pre-trained model to obtain a detection result; and enter the retention detection mode when the detection result indicates whether the endoscopic examination is completed.

In one or more embodiments, the apparatus further includes a mode determination module, configured to determine whether a wake-up duration of the capsule endoscope has reached a preset duration, and enter the retention detection mode in response to a wake-up duration of the capsule endoscope reaching a preset duration.

In one or more embodiments, the wake-up module is further configured to trigger the counter to count cyclically according to the clock signal generated by the clock signal generator, wherein the number of counting cycles is configured to determine whether the counter overflows; trigger the counter to count according to the clock signal generated by the clock signal generator during each counting operation in a cyclic process; and reset the count value for a next counting cycle when the count of the counter reaches a preset value.

In one or more embodiments, the apparatus further includes a switch control module, configured to turn off the counter after the radio-frequency transmission circuit is woken up; and trigger the counter to resume counting after the command is issued.

In one or more embodiments, the retention determination module is further configured to determine that the capsule endoscope is retained in the examinee's body when the data recorder receives the command; and determine that the capsule endoscope has been excreted from the examinee's body when the data recorder does not receive the command within a preset time duration.

In one or more embodiments, the apparatus further includes an image sending module, configured to wake up an image sensor and a main control chip of the capsule endoscope to capture an image when determining that the capsule endoscope is retained in the examinee's body; and send the image to the data recorder, wherein the image is configured to determine a cause of retention of the capsule endoscope.

In a third aspect, the present invention further provides a capsule endoscope, wherein the capsule endoscope includes a counter, a radio-frequency transmission circuit, a clock signal generator, and a register, wherein
the register is configured to store a command;
the clock signal generator is configured to generate a clock signal;
the counter is configured to count;
in a retention detection mode, other modules in the capsule endoscope except the counter, the clock signal generator, and the register is powered off, and the radio-frequency transmission circuit is intermittently woken up to issue a command, wherein
the radio-frequency transmission circuit is woken up when the counter overflows.

In a fourth aspect, the present invention further provides a computer-readable storage medium. The computer-readable storage medium has stored thereon a computer program which, when executed by a processor, causes the processor to perform the above method.

In a fifth aspect, the present invention further provides a computer program product. The computer program product includes a computer program which, when executed by a processor, causes the processor to perform the above method.

The embodiments of the present invention can achieve the following beneficial technical effects. In the method for detecting excretion of a capsule endoscope, the apparatus, the capsule endoscope, the storage medium, and the computer program product, in a retention detection mode, other modules in the capsule endoscope except the counter, the clock signal generator, and the register is powered off, and the radio-frequency transmission circuit is intermittently woken up to issue a command, wherein the command is pre-stored in the register; the counter is triggered to count according to the clock signal generated by the clock signal generator during each wake-up process, and when the counter overflows, the radio-frequency transmission circuit is woken up to issue the command; and it is determined that the capsule endoscope is retained in an examinee's body in response to a data recorder receiving the command. The present solution turns off the power supply of other modules in the capsule endoscope except the counter, the clock signal generator, and the register, eliminating the need to supply power to other modules, thereby reducing power consumption. Moreover, by intermittently waking up the radio-frequency transmission circuit to issue the command, power is supplied to the radio-frequency transmission circuit only when it is woken up, further reducing power consumption, thus extending the operation duration of the capsule endoscope and improving the detection rate of capsule endoscope retention.

The embodiments of the present invention are further described below based on the drawings of the present invention.

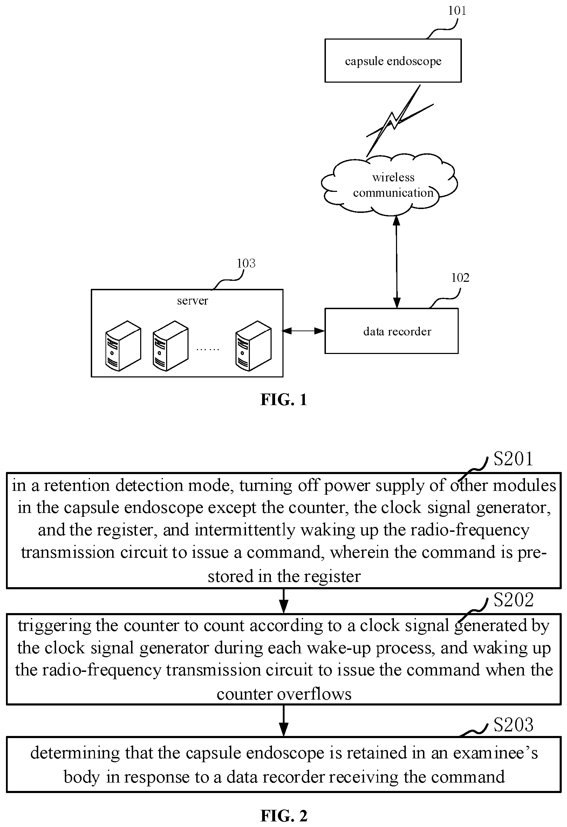

The method for detecting excretion of a capsule endoscope provided by the embodiments of the present invention can be applied in the application environment shown in FIG. 1. Here, a capsule endoscope 101 performs data exchange with a data recorder 102 through wireless communication, which can include radio-frequency communication. A server 103 can process data received by the data recorder 102 to determine whether the capsule endoscope 101 has been excreted from the examinee's body. In the retention detection mode, the power supply of other modules in the capsule endoscope 101 except the counter, the clock signal generator, and the register is turned off, and the radio-frequency transmission circuit is intermittently woken up to issue a command, wherein the command is pre-stored in the register; during each wake-up process, the counter is triggered to count according to the clock signal generated by the clock signal generator, and when the counter overflows, the radio-frequency transmission circuit is woken up to issue the command; and it can be determined that the capsule endoscope is retained in an examinee's body in response to a data recorder 102 receiving the command. Here, the data recorder 102 includes a radio-frequency receiving module for receiving the above command. The data recorder 102 can be, but is not limited to, various personal computers, laptops, smartphones, tablets, Internet of Things devices, and portable wearable devices. The Internet of Things devices can include smart speakers, smart TVs, smart air conditioners, smart in-vehicle devices, etc. The portable wearable devices can include smart watches, smart bands, head-mounted devices, etc.

In an exemplary embodiment, as shown in FIG. 2, a method for detecting excretion of a capsule endoscope is provided, described by taking the method applied to the capsule endoscope 101 in FIG. 1 as an example, and the method includes the following steps S201 to S203.

Step S201: in a retention detection mode, powering off other modules in the capsule endoscope except the counter, the clock signal generator, and the register, and intermittently waking up the radio-frequency transmission circuit to issue a command, wherein the command is pre-stored in the register.

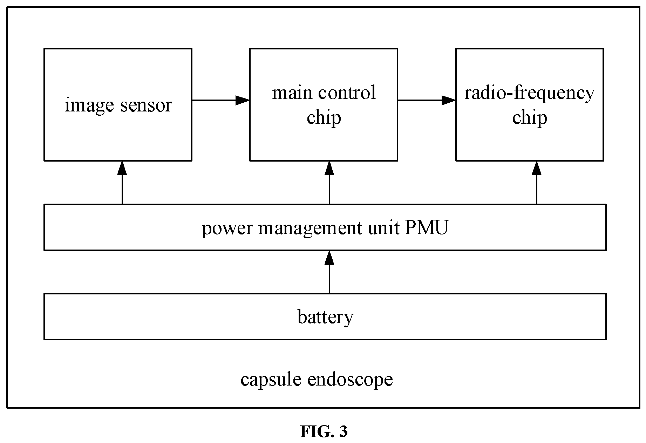

An internal structure of the capsule endoscope is shown in FIG. 3. The capsule endoscope includes an image sensor, a main control chip, a radio-frequency chip (which can be referred to as an RF chip, where RF stands for Radio Frequency), a battery, and a power management unit. The power management unit can be implemented by a power management unit (PMU). The main control chip is connected to the image sensor and is configured to capture images and process data. The radio-frequency chip includes a logic control module, a radio-frequency transmission circuit, a register, a counter, and a clock signal generator, and is configured to send images or commands to a device outside the examinee's body, such as a data recorder. The external data recorder processes the received data. The register can include a First-In-First-Out (FIFO) register.

When the capsule endoscope completes detection of a designated site in the digestive tract, the capsule endoscope enters the retention detection mode. In the above retention detection mode, the power management unit can turn off the power supply of other modules in the capsule endoscope except the counter, the clock signal generator, and the register, and the radio-frequency transmission circuit is intermittently woken up to issue a command pre-stored in the register. The specific steps of intermittently waking up the radio-frequency transmission circuit to issue the command are as follows: waking up the radio-frequency transmission circuit to issue the command each time the counter overflows. Specifically, after the counter overflows once, the radio-frequency transmission circuit is woken up to issue the command, and after the command is issued, the counter starts the next counting. When the next counting overflows, the radio-frequency transmission circuit is woken up again to issue the command, and this process is repeated.

In the retention detection mode, when the counter of the radio-frequency chip counts according to the clock signal generated by the clock signal generator, other components of the radio-frequency chip except the counter and the clock signal generator are not working and are in a sleep state. Thus, the radio-frequency chip in the retention detection mode can be considered to be in a sleep counting state (also referred to as a deep sleep state). The radio-frequency chip operates in a narrow pulse mode, and power is supplied only when the radio-frequency transmission circuit is woken up. The rest of the time, it is in the sleep counting state, where the supply current is less than 1.5 µA, resulting in low power consumption.

The clock signal generator generates a clock signal below 1 KHz by frequency-dividing an external low-speed passive crystal oscillator clock. The counter is configured to count according to the clock signal. The register is configured to pre-store a command, which can be referred to as a retention identification command and can be implemented by a specific communication command. The radio-frequency transmission circuit is configured to issue the command. The command has a small amount of data, and the transmission duration of the radio-frequency transmission circuit is short, further saving power consumption.

In this step, by intermittently waking up the radio-frequency transmission circuit to issue the command via the counter, compared to the conventional method of continuously sending signals to the data recorder, there is no need to supply power to other modules except the counter, the clock signal generator, and the register. Power is supplied only when the radio-frequency transmission circuit issues the command, saving power consumption.

Step S202: triggering the counter to count according to a clock signal generated by the clock signal generator during each wake-up process, and waking up the radio-frequency transmission circuit to issue the command when the counter overflows.

One process from the counter starting to count to the radio-frequency transmission circuit issuing the command can be referred to as one wake-up process. In each wake-up process, the logic control module triggers the counter to count according to the clock signal generated by the clock signal generator. When the counter overflows, the counter issues a trigger signal. Only after the trigger signal is issued, power is supplied to the radio-frequency transmission circuit, so that the radio-frequency transmission circuit is woken up in response to the trigger signal. The radio-frequency transmission circuit then enters the command transmission state in the retention detection mode, which means that the radio-frequency transmission circuit transmits the command pre-stored in the register to the external data recorder.

Step S203: determining that the capsule endoscope is retained in an examinee's body in response to a data recorder receiving the command.

The data recorder can include a radio-frequency receiving module that can receive and process the command. The data recorder can be within a set spatial range centered on the examinee. If the data recorder is outside the set spatial range, regardless of whether the capsule endoscope has been excreted, the command issued by the radio-frequency transmission circuit of the capsule endoscope can never be received by the data recorder, making excretion detection difficult. Specifically, the data recorder can be worn on the examinee's body. This ensures that whether the capsule endoscope is retained in the examinee's body can be determined based on whether the data recorder receives the command.

After the data recorder is worn on the examinee's body, if the data recorder does not receive the command within a preset time duration, it indicates that the distance between the data recorder and the capsule endoscope is large, and the capsule endoscope has been excreted from the examinee's body. If the data recorder receives the command, it indicates that the distance between the data recorder and the capsule endoscope is small, and the capsule endoscope has not been excreted from the examinee's body; and in this case, it can be determined that the capsule endoscope is retained in the examinee's body. In the case where it is determined that the capsule endoscope is retained in the examinee's body, the radio-frequency transmission circuit can continue to be intermittently woken up to issue the command.

In the above method for detecting excretion of a capsule endoscope, the power supply of other modules in the capsule endoscope except the counter, the clock signal generator, and the register is turned off, eliminating the need to supply power to other modules, thereby reducing power consumption. Moreover, by intermittently waking up the radio-frequency transmission circuit to issue the command, power is supplied to the radio-frequency transmission circuit only when it is woken up, further reducing power consumption, thus extending the operation duration of the capsule endoscope and improving the detection rate of capsule endoscope retention. Furthermore, the present solution can help the examinee promptly detect whether the capsule endoscope has been excreted from the body, without the need for the examinee to go to a hospital for X-ray irradiation, thereby avoiding radiation damage. It provides another radiation-free detection method for examinees who cannot undergo radiation detection, allowing the examinee to self-confirm whether the capsule endoscope is retained, so as to report to the system at the first opportunity to obtain the best timing for resolution, thereby improving the convenience and safety of capsule endoscope detection.

In one or more embodiments, before step S201, i.e., before a step of in a retention detection mode, powering off other modules in the capsule endoscope except the counter, the clock signal generator, and the register, and intermittently waking up the radio-frequency transmission circuit to issue a command, the method provided by the present invention further includes: determining whether a user issued endoscopic examination completion command is received; and entering the retention detection mode in response to receiving a user issued endoscopic examination completion command.

When the endoscopic examination completion command from the user is received, it indicates that the user, through an external display device, determines that the capsule endoscope has completed the digestive tract examination. At this time, the logic control module determines that the capsule endoscope can enter the retention detection mode. When the endoscopic examination completion command from the user is not received, it indicates that the user, through the external display device, determines that the capsule endoscope has not yet completed the digestive tract examination. At this time, the logic control module determines that the capsule endoscope is not suitable to enter the retention detection mode and should remain in the working state to continue examining the digestive tract.

In this embodiment, based on whether the endoscopic examination completion command from the user is received, the logic control module determines whether the capsule endoscope can enter the retention detection mode. When the endoscopic examination completion command from the user is received, the logic control module determines that the capsule endoscope can enter the retention detection mode, thereby increasing the probability that the capsule endoscope has completed the digestive tract examination.

In one or more embodiments, before step S201, i.e., before a step of in the retention detection mode, powering off other modules in the capsule endoscope except the counter, the clock signal generator, and the register, and intermittently waking up the radio-frequency transmission circuit to issue a command, the method provided by the present invention further includes: acquiring a current image captured by a camera; inputting the current image into a pre-trained model to obtain a detection result; and entering the retention detection mode when the detection result indicates whether the endoscopic examination is completed.

The image sensor and the main control chip of the capsule endoscope can control the camera of the capsule endoscope to capture an image, and input the captured image into a pre-trained model, which can be built by Artificial Intelligence (AI), so as to obtain a detection result. When the detection result indicates whether the endoscopic examination is completed (i.e., digestive tract examination), the capsule endoscope enters the retention detection mode. The above process of inputting the captured image into the model to obtain a detection result and then determining whether the capsule endoscope can enter the retention detection mode based on the detection result can be automatically completed by artificial intelligence.

In this embodiment, a detection result of whether the endoscopic examination is completed is obtained based on the captured image, and then it is determined whether the capsule endoscope can enter the retention detection mode based on the detection result. When the detection result indicates whether the endoscopic examination is completed, the capsule endoscope automatically enters the retention detection mode, without the need to determine whether the digestive tract examination is completed based on whether the user has issued an endoscopic examination completion command. Artificial Intelligence can automatically determine whether the digestive tract examination is completed, thereby determining whether the capsule endoscope can enter the retention detection mode.

In one or more embodiments, before step S201, i.e., before a step of in the retention detection mode, powering off other modules in the capsule endoscope except the counter, the clock signal generator, and the register, and intermittently waking up the radio-frequency transmission circuit to issue a command, the method provided by the present invention further includes: determining whether a wake-up duration of the capsule endoscope has reached a preset duration; and entering the retention detection mode in response to a wake-up duration of the capsule endoscope reaching a preset duration.

The preset duration can be set according to actual conditions. After the examinee wakes up the capsule endoscope and swallows it, the capsule endoscope sequentially passes through the esophagus, stomach, duodenum, jejunum and ileum, colon, and rectum, and is finally excreted. The entire examination generally takes 6 to 8 hours. Within 8 to 72 hours after swallowing, the capsule endoscope is excreted with feces. Therefore, the preset duration can be set to 8 hours.

In this embodiment, when it is determined that the wake-up duration of the capsule endoscope has reached the preset duration, it is highly probable that the capsule endoscope has completed the digestive tract examination. At this time, the logic control module determines that the capsule endoscope can enter the retention detection mode, without the need to determine whether the digestive tract examination is completed based on whether the user has issued an endoscopic examination completion command, nor the need for Artificial Intelligence to automatically determine whether the digestive tract examination is completed.

In one or more embodiments, the step of triggering the counter to count according to a clock signal generated by the clock signal generator in step S202 includes the following specific steps: triggering the counter to count cyclically according to the clock signal generated by the clock signal generator, wherein the number of counting cycles is configured to determine whether the counter overflows; and triggering the counter to count according to the clock signal generated by the clock signal generator during each counting operation in a cyclic process, and resetting the count value for a next counting cycle when the count of the counter reaches a preset value.

The preset count value can be set according to actual conditions.

The logic control module triggers the counter to count according to the clock signal generated by the clock signal generator. The clock signal can be in minutes. When the count of the counter reaches the preset count value, the count value is reset, and the next counting starts. Each time the next counting starts, the number of counting cycles increases by one. The above counting process is repeated until the number of counting cycles reaches a preset cycle value, at which point it is determined that the counter overflows.

Exemplarily, when the count of the counter reaches a preset count value of 60, the count value is reset, and the next counting starts. At this time, the number of counting cycles is 1. When the count of the counter reaches the preset count value of 60 again, the count value is reset, and the next counting starts. At this time, the number of counting cycles is 2. The above counting process is repeated until the number of counting cycles reaches a preset cycle value of 4, at which point it is determined that the counter overflows.

In this embodiment, the logic control module triggers the counter to count cyclically according to the clock signal generated by the clock signal generator, so the count value is small, reducing the memory occupied by counting.

In one or more embodiments, after waking up the radio-frequency transmission circuit in step S202, the method provided by the present invention further includes: turning off the counter after the radio-frequency transmission circuit is woken up; and triggering the counter to resume counting after the command is issued.

In this embodiment, after the counter wakes up the radio-frequency transmission circuit, the counter is turned off; and after the radio-frequency transmission circuit issues the command, the logic control module triggers the counter to resume counting, further reducing the power consumption of the capsule endoscope.

In one or more embodiments, the step of determining that the capsule endoscope is retained in an examinee's body in response to a data recorder receiving the command in step S203 includes the following specific steps: determining that the capsule endoscope is retained in the examinee's body when the data recorder receives the command; or determining that the capsule endoscope has been excreted from the examinee's body when the data recorder does not receive the command within a preset time duration.

The data recorder includes a radio-frequency receiving module that can receive the command issued by the radio-frequency transmission circuit.

If the data recorder does not receive the command within a preset time duration, it indicates that the distance between the data recorder and the capsule endoscope is large, and the capsule endoscope has been excreted from the examinee's body. If the data recorder receives the command, it indicates that the distance between the data recorder and the capsule endoscope is small, and the capsule endoscope has not been excreted from the examinee's body; and in this case, it can be determined that the capsule endoscope is retained in the examinee's body. In the case where it is determined that the capsule endoscope is retained in the examinee's body, the radio-frequency transmission circuit can continue to be intermittently woken up to issue the command.

Further, a command number threshold can be set according to actual conditions. When the number of consecutive times the data recorder receives the command exceeds the command number threshold, it can be further determined that the capsule endoscope is retained in the examinee's body, thereby improving the accuracy of determining that the capsule endoscope is retained in the examinee's body.

In this embodiment, it is determined whether the capsule endoscope is retained in the examinee's body based on whether the data recorder receives the command.

In one or more embodiments, after the step of determining that the capsule endoscope is retained in an examinee's body in response to a data recorder receiving the command, the method provided by the present invention further includes: waking up an image sensor and a main control chip of the capsule endoscope to capture an image when determining that the capsule endoscope is retained in the examinee's body; and sending the image to the data recorder, wherein the image is configured to determine a cause of retention of the capsule endoscope.

After it is determined that the capsule endoscope is retained in the examinee's body, a user such as a doctor or the examinee can send an external command (which can be referred to as an external control command). When the capsule endoscope receives the external command, it wakes up the image sensor and the main control chip to capture an image. The command control logic circuit inside the radio-frequency chip sends the above image to the data recorder. The server can process the above image received by the data recorder and draw relevant conclusions, so that the user can determine the cause of retention of the capsule endoscope according to the above image.

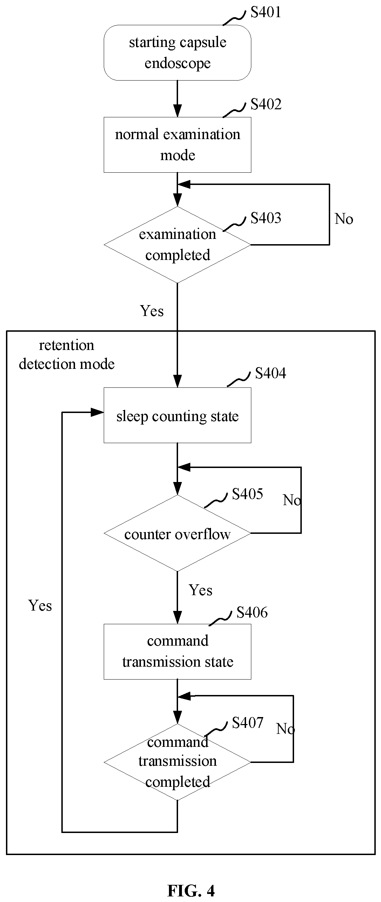

To better understand the above method, an application embodiment of the method for detecting excretion of a capsule endoscope is described in detail below. The process flow of this application embodiment is shown in FIG. 4, including steps S401~S407.

Step S401: starting the capsule endoscope. Step S402: the capsule endoscope entering a normal examination mode to examine the digestive tract of the examinee. Step S403: determining whether the digestive tract examination is completed, if the capsule endoscope has not completed the digestive tract examination, continuing the digestive tract examination; otherwise, proceeding to step S404. Step S404: entering the retention detection mode, turning off the power supply of other modules in the capsule endoscope except the counter, the clock signal generator, and the register, and causing the radio-frequency chip to enter a sleep counting state, in which the counter counts according to the clock signal generated by the clock signal generator. Step S405: determining whether the counter overflows, if so, proceeding to step S406. Step S406: the radio-frequency transmission circuit entering the command transmission state in the retention detection mode and transmitting the command pre-stored in the register to the external data recorder. Step S407: the logic control module determining whether the command transmission is completed, if the command transmission is completed, the radio-frequency chip re-entering the sleep counting state, and the counter resuming counting according to the clock signal generated by the clock signal generator.

In the above embodiment, the power supply of other modules in the capsule endoscope except the counter, the clock signal generator, and the register is turned off, eliminating the need to supply power to other modules, thereby reducing power consumption. Moreover, by intermittently waking up the radio-frequency transmission circuit to issue the command, power is supplied to the radio-frequency transmission circuit only when it is woken up, further reducing power consumption, thus extending the operation duration of the capsule endoscope and improving the detection rate of capsule endoscope retention.

It should be understood that although the steps in the flowcharts of the above-described embodiments are shown sequentially as indicated by arrows, these steps are not necessarily performed sequentially in the order indicated by the arrows. Unless explicitly stated herein, there is no strict order restriction on the execution of these steps, and they can be performed in other orders. Moreover, at least some of the steps in the flowcharts of the above-described embodiments can include a plurality of steps or stages. These steps or stages are not necessarily completed at the same time but can be performed at different times, and the order of execution of these sub-steps or stages is not necessarily sequential but can be performed alternately or interchangeably with at least a part of other steps or sub-steps or stages of other steps.

Based on the same inventive concept, the embodiments of the present invention further provide an apparatus of detecting excretion of a capsule endoscope, which is configured to implement the above-described method for detecting excretion of a capsule endoscope. The implementation provided by the apparatus is similar to that described in the above method. Therefore, for specific details of one or more embodiments of the apparatus of detecting excretion of a capsule endoscope provided below, reference can be made to the above description of the method for detecting excretion of a capsule endoscope, and details will not be repeated here.

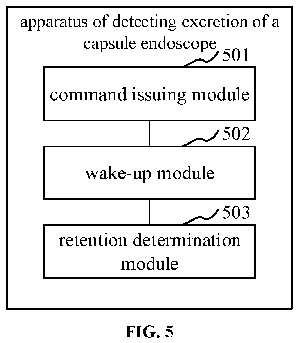

In an exemplary embodiment, as shown in FIG. 5, an apparatus of detecting excretion of a capsule endoscope is provided. The capsule endoscope includes a counter, a radio-frequency transmission circuit, a clock signal generator, and a register. The apparatus includes:
a command issuing module 501, configured to in a retention detection mode, power off other modules in the capsule endoscope except the counter, the clock signal generator, and the register, and intermittently wake up the radio-frequency transmission circuit to issue a command, wherein the command is pre-stored in the register;
a wake-up module 502, configured to trigger the counter to count according to a clock signal generated by the clock signal generator during each wake-up process, and wake up the radio-frequency transmission circuit to issue the command when the counter overflows; and
a retention determination module 503 configured to determine that the capsule endoscope is retained in an examinee's body in response to a data recorder receiving the command.

In one or more embodiments, the apparatus further includes a mode determination module, configured to determine whether a user issued endoscopic examination completion command is received, and enter the retention detection mode in response to receiving a user issued endoscopic examination completion command issued.

In one or more embodiments, the apparatus further includes a mode determination module, configured to acquire a current image captured by a camera; input the current image into a pre-trained model to obtain a detection result; and enter the retention detection mode when the detection result indicates whether the endoscopic examination is completed.

In one or more embodiments, the apparatus further includes a mode determination module, configured to determine whether a wake-up duration of the capsule endoscope has reached a preset duration, and enter the retention detection mode in response to a wake-up duration of the capsule endoscope reaching a preset duration.

In one or more embodiments, the wake-up module 502 is further configured to trigger the counter to count cyclically according to the clock signal generated by the clock signal generator, wherein the number of counting cycles is configured to determine whether the counter overflows; trigger the counter to count according to the clock signal generated by the clock signal generator during each counting operation in a cyclic process; and reset the count value for a next counting cycle when the count of the counter reaches a preset value.

In one or more embodiments, the apparatus further includes a switch control module, configured to turn off the counter after the radio-frequency transmission circuit is woken up; and trigger the counter to resume counting after the command is issued.

In one or more embodiments, the retention determination module 503 is further configured to determine that the capsule endoscope is retained in the examinee's body when the data recorder receives the command; and determine that the capsule endoscope has been excreted from the examinee's body when the data recorder does not receive the command within a preset time duration.

In one or more embodiments, the apparatus further includes an image sending module, configured to wake up an image sensor and a main control chip of the capsule endoscope to capture an image when determining that the capsule endoscope is retained in the examinee's body; and send the image to the data recorder, wherein the image is configured to determine a cause of retention of the capsule endoscope.

Each module in the above apparatus of detecting excretion of a capsule endoscope can be implemented in whole or in part by software, hardware, or a combination thereof. The above modules can be embedded in or independent of the processor of the capsule endoscope in hardware form, or can be stored in the memory of the capsule endoscope in software form, so that the processor can invoke and execute the operations corresponding to the above modules.

In one embodiment, a capsule endoscope is further provided. The capsule endoscope includes a counter, a radio-frequency transmission circuit, a clock signal generator, and a register, wherein the register is configured to store a command; the clock signal generator is configured to generate a clock signal; the counter is configured to count, wherein in a retention detection mode, other modules in the capsule endoscope except the counter, the clock signal generator, and the register is powered off, and the radio-frequency transmission circuit is intermittently woken up to issue a command, wherein the radio-frequency transmission circuit is woken up when the counter overflows.

The capsule endoscope of this embodiment can also perform the steps of any one of the method embodiments, which will not be repeated here.

In one embodiment, a computer-readable storage medium storing a computer program is provided, wherein the computer program, when executed by a processor, causes the processor to perform the steps in the above method embodiments.

In one embodiment, a computer program product is provided, including a computer program which, when executed by a processor, causes the processor to perform the steps in the above method embodiments.

It should be noted that the user information (including but not limited to user device information, user personal information, etc.) and data (including but not limited to data for analysis, stored data, displayed data, etc.) involved in the present invention are all information and data that have been authorized by the user or fully authorized by all parties, and the collection, use, and processing of relevant data must comply with relevant regulations.

Those of ordinary skill in the art will understand that all or part of the processes in the methods of the above embodiments can be implemented by using a computer program to instruct relevant hardware. The computer program can be stored in a non-volatile computer-readable storage medium, and when executed, the computer program can perform the processes of the above method embodiments. Any reference to a memory, a database, or other medium used in the embodiments provided in the present invention can include at least one of non-volatile and volatile memory. Non-volatile memory can include Read-Only Memory (ROM), magnetic tape, floppy disk, flash memory, optical memory, high-density embedded non-volatile memory, Resistive Memory (ReRAM), Magnetoresistive Random Access Memory (MRAM), Ferroelectric Random Access Memory (FRAM), Phase Change Memory (PCM), graphene memory, and the like. Volatile memory can include Random Access Memory (RAM) or external cache memory, and the like. By way of illustration and not limitation, RAM can be in various forms, such as Static Random Access Memory (SRAM) or Dynamic Random Access Memory (DRAM), and the like. The databases involved in the embodiments provided in the present disclosure can include at least one of relational databases and non-relational databases. Non-relational databases may include a blockchain-based distributed database, and the like, but are not limited thereto.

The technical features of the above embodiments can be combined arbitrarily. For the sake of brevity, not all possible combinations of the technical features in the above embodiments are described. However, as long as there is no contradiction in the combination of these technical features, it should be considered to fall within the scope of this description.

The above embodiments are only several implementations of the present invention, and their descriptions are relatively specific and detailed, but they should not be construed as limiting the scope of the present invention. It should be noted that, for those of ordinary skill in the art, various modifications and improvements can be made without departing from the concept of the present invention, and such modifications and improvements shall fall within the protection scope of the present invention. Therefore, the protection scope of the present invention shall be subject to the appended claims.

### INDUSTRIAL APPLICABILITY

In the above method for detecting excretion of a capsule endoscope, the apparatus, the capsule endoscope, the storage medium, and the computer program product, in a retention detection mode, other modules in the capsule endoscope except the counter, the clock signal generator, and the register is powered off, and the radio-frequency transmission circuit is intermittently woken up to issue a command, wherein the command is pre-stored in the register; during each wake-up process, the counter is triggered to count according to the clock signal generated by the clock signal generator, and when the counter overflows, the radio-frequency transmission circuit is woken up to issue the command; and whether the capsule endoscope is retained in the examinee's body is determined according to whether the data recorder receives the command. The present solution turns off the power supply of other modules in the capsule endoscope except the counter, the clock signal generator, and the register, eliminating the need to supply power to other modules, thereby reducing power consumption. Moreover, by intermittently waking up the radio-frequency transmission circuit to issue the command, power is supplied to the radio-frequency transmission circuit only when it is woken up, further reducing power consumption, thus extending the operation duration of the capsule endoscope and improving the detection rate of capsule endoscope retention.

In addition, the method for detecting excretion of a capsule endoscope, the apparatus, and the capsule endoscope provided by the present invention are reproducible and applicable to various technical fields. For example, the method for detecting excretion of a capsule endoscope, the apparatus, and the capsule endoscope provided by the present invention can be applied to the technical field of capsule endoscope detection.

## Claims

1. A method for detecting excretion of a capsule endoscope, **characterized in that** the capsule endoscope comprises a counter, a radio-frequency transmission circuit, a clock signal generator, and a register; and the method comprises:
in a retention detection mode, powering off other modules in the capsule endoscope except the counter, the clock signal generator, and the register, and intermittently waking up the radio-frequency transmission circuit to issue a command, wherein the command is pre-stored in the register;
triggering the counter to count according to a clock signal generated by the clock signal generator during each wake-up process, and waking up the radio-frequency transmission circuit to issue the command when the counter overflows; and
determining that the capsule endoscope is retained in an examinee's body in response to a data recorder receiving the command.

2. The method according to claim 1, wherein before a step of in a retention detection mode, powering off other modules in the capsule endoscope except the counter, the clock signal generator, and the register, and intermittently waking up the radio-frequency transmission circuit to issue a command, the method further comprises:
determining whether a user issued endoscopic examination completion command is received; and
entering the retention detection mode in response to receiving a user issued endoscopic examination completion command.

3. The method according to claim 1, wherein before a step of in a retention detection mode, powering off other modules in the capsule endoscope except the counter, the clock signal generator, and the register, and intermittently waking up the radio-frequency transmission circuit to issue a command, the method further comprises:
acquiring a current image captured by a camera;
inputting the current image into a pre-trained model to obtain a detection result; and
entering the retention detection mode when the detection result indicates whether the endoscopic examination is completed.

4. The method according to claim 1, wherein before a step of in a retention detection mode, powering off other modules in the capsule endoscope except the counter, the clock signal generator, and the register, and intermittently waking up the radio-frequency transmission circuit to issue a command, the method further comprises:
determining whether a wake-up duration of the capsule endoscope has reached a preset duration; and
entering the retention detection mode in response to a wake-up duration of the capsule endoscope reaching a preset duration.

5. The method according to claim 1, wherein a step of triggering the counter to count according to a clock signal generated by the clock signal generator comprises:
triggering the counter to count cyclically according to the clock signal generated by the clock signal generator, wherein the number of counting cycles is configured to determine whether the counter overflows; and
triggering the counter to count according to the clock signal generated by the clock signal generator during each counting operation in a cyclic process, and resetting the count value for a next counting cycle when the count of the counter reaches a preset value.

6. The method according to claim 1, wherein after waking up the radio-frequency transmission circuit, the method further comprises:
turning off the counter after the radio-frequency transmission circuit is woken up; and
triggering the counter to resume counting after the command is issued.

7. The method according to claim 1, wherein a step of determining that the capsule endoscope is retained in an examinee's body in response to a data recorder receiving the command comprises:
determining that the capsule endoscope is retained in the examinee's body when the data recorder receives the command; or
determining that the capsule endoscope has been excreted from the examinee's body when the data recorder does not receive the command within a preset time duration.

8. The method according to claim 1, wherein after determining that the capsule endoscope is retained in an examinee's body in response to a data recorder receiving the command, the method further comprises:
waking up an image sensor and a main control chip of the capsule endoscope to capture an image when determining that the capsule endoscope is retained in the examinee's body; and
sending the image to the data recorder, wherein the image is configured to determine a cause of retention of the capsule endoscope.

9. An apparatus of detecting excretion of a capsule endoscope, **characterized in that** the capsule endoscope comprises a counter, a radio-frequency transmission circuit, a clock signal generator, and a register; and the apparatus comprises:
a command issuing module, configured to in a retention detection mode, power off other modules in the capsule endoscope except the counter, the clock signal generator, and the register, and intermittently wake up the radio-frequency transmission circuit to issue a command, wherein the command is pre-stored in the register;
a wake-up module, configured to trigger the counter to count according to a clock signal generated by the clock signal generator during each wake-up process, and wake up the radio-frequency transmission circuit to issue the command when the counter overflows; and
a retention determination module, configured to determine that the capsule endoscope is retained in an examinee's body in response to a data recorder receiving the command.

10. The apparatus of detecting excretion of a capsule endoscope according to claim 9, wherein the apparatus further comprises a mode determination module, configured to determine whether a user issued endoscopic examination completion command is received, and enter the retention detection mode in response to receiving a user issued endoscopic examination completion command.

11. The apparatus of detecting excretion of a capsule endoscope according to claim 9, wherein the apparatus further comprises a mode determination module, configured to acquire a current image captured by a camera; input the current image into a pre-trained model to obtain a detection result; and enter the retention detection mode when the detection result indicates whether the endoscopic examination is completed.

12. The apparatus of detecting excretion of a capsule endoscope according to claim 9, wherein the apparatus further comprises a mode determination module, configured to determine whether a wake-up duration of the capsule endoscope has reached a preset duration, and enter the retention detection mode in response to a wake-up duration of the capsule endoscope reaching a preset duration.

13. The apparatus of detecting excretion of a capsule endoscope according to claim 9, wherein the wake-up module is further configured to trigger the counter to count cyclically according to the clock signal generated by the clock signal generator, wherein the number of counting cycles is configured to determine whether the counter overflows; trigger the counter to count according to the clock signal generated by the clock signal generator during each counting operation in a cyclic process; and reset the count value for a next counting cycle when the count of the counter reaches a preset value.

14. The apparatus of detecting excretion of a capsule endoscope according to claim 9, wherein the apparatus further comprises a switch control module, configured to turn off the counter after the radio-frequency transmission circuit is woken up, and trigger the counter to resume counting after the command is issued.

15. The apparatus of detecting excretion of a capsule endoscope according to claim 9, wherein the retention determination module is further configured to determine that the capsule endoscope is retained in the examinee's body when the data recorder receives the command; and determine that the capsule endoscope has been excreted from the examinee's body when the data recorder does not receive the command within a preset time duration.

16. The apparatus of detecting excretion of a capsule endoscope according to claim 9, wherein the apparatus further comprises an image sending module, configured to wake up an image sensor and a main control chip of the capsule endoscope to capture an image when determining that the capsule endoscope is retained in the examinee's body; and send the image to the data recorder, wherein the image is configured to determine a cause of retention of the capsule endoscope.

17. A capsule endoscope, wherein the capsule endoscope comprises a counter, a radio-frequency transmission circuit, a clock signal generator, and a register, wherein
the register is configured to store a command;
the clock signal generator is configured to generate a clock signal;
the counter is configured to count, wherein
in a retention detection mode, other modules in the capsule endoscope except the counter, the clock signal generator, and the register is powered off, and the radio-frequency transmission circuit is intermittently woken up to issue a command, wherein
the radio-frequency transmission circuit is woken up when the counter overflows.

18. A computer-readable storage medium storing a computer program which, when executed by a processor, causes the processor to perform the steps of the method according to any one of claims 1 to 8.

19. A computer program product comprising a computer program which, when executed by a processor, causes the processor to perform the steps of the method according to any one of claims 1 to 8.
